# EUROPÄISCHE PATENTANMELDUNG

(11) **EP 2 452 621 A1**
(43) Veröffentlichungstag der Anmeldung: **16.05.2012**
(21) Anmeldenummer: 11450144.8
(22) Anmeldetag: 16.11.2011
(51) Int. Cl.: A61B 5/0452

(54) **Verfahren zur Beurteilung des kardiovaskulären Status einer Person durch EKG-Messung während künstlicher Erhöhung des Widerstandes von Blutgefäßen**

(30) Priorität: 16.11.2010 AT 18842010
(71) Anmelder: AIT Austrian Institute of Technology GmbH, 1220 Wien (AT)
(72) Erfinder: Hayn, Dieter, 8010 Graz (AT); Schreier, Günther, 8010 Graz (AT)
(74) Vertreter: Wildhack & Jellinek

(57) **Zusammenfassung**

Das Patent betrifft ein Verfahren zur Beurteilung des kardiovaskulären Status einer Person (11) durch Bestimmung spezifischer Kenngrößen des EKGs, wobei während der EKG-Messung z.B. mit einer Manschette (12), die über einen Luftschlauch (13) mit einem Blutdruckmessgerät (14) verbunden ist, der Gefäßwiderstand mindestens eines Blutgefäßes dieser Person künstlich erhöht wird und mit einem EKG-Rekorder (15), der über Kable (16) mit an der Person angebrachten Elektroden (17) verbunden ist, ein EKG aufgezeichnet und die Kenngrößen des EKGs bei erhöhtem und bei normaler Gefäßwiderstand in einer Auswerteeinheit (18) miteinander verglichen werden.

## Beschreibung

Das Patent betrifft ein Verfahren zur Beurteilung des kardiovaskulären Status einer Person durch Bestimmung spezifischer Kenngrößen des Elektrokardiogramms (EKG).

Die Analyse des EKGs ist heute ein wichtiger Bestandteil der medizinischen Diagnostik insbesondere von Erkrankungen des Herz-Kreislauf-Systems und es gibt bereits zahlreiche Kenngrößen, die zur Diagnose eingesetzt werden oder deren Einsatz derzeit erforscht wird.

Ein weiteres Verfahren zur Diagnose von Erkrankungen des Herz-Kreislauf-Systems ist die Messung des Blutdrucks. Das hierbei am häufigsten verwendete Verfahren ist jenes nach Riva-Rocci, während dem die Blutzufuhr zu einer Hand mit Hilfe einer aufblasbaren Manschette unterbunden wird.

Stand der Technik sind auch zahlreiche Verfahren, in denen Blutdruck und EKG gleichzeitig gemessen werden und die Parameter der beiden Messungen (hämodynamische Parameter und elektrophysiologische Parameter) miteinander in Verbindung gesetzt werden. Derartige Verfahren untersuchen zwar den Zusammenhang zwischen Blutdruck und EKG, nicht aber die Auswirkungen der Blutdruckmessung selbst auf das EKG.

Durch die Unterbrechung der Blutzufuhr zu einer Extremität (z.B. Arm) wird der Regelkreis des Herz-Kreislauf-Systems empfindlich gestört: Der Gefäßwiderstand dieser Extremität erhöht sich, das Körpervolumen, das vom Herzen mit Blut versorgt werden kann, wird reduziert, die Menge des rückströmenden Blutes geht zurück, die Gesamtkapazität der Gefäße wird verringert, etc.

Jede Störung im Regelkreis wird vom Körper durch eine Veränderung der Herzaktivität und/oder der Gefäßeigenschaften ausgeglichen, die wiederum im EKG beobachtet werden kann. Wie deutlich die Änderungen im EKG bei einer Störung des Regelkreises ausfallen, hängt von zahlreichen Faktoren ab, wie z.B. von der Anpassungsfähigkeit des Herzens und des gesamten autonomen Nervensystems oder von den mechanischen Eigenschaften der Gefäße (z.B. Grad der Verkalkung der Gefäße).

Der Erfindung liegt also die Aufgabe zugrunde, die Eigenschaften des Herz-Kreislauf-Systems und/oder die Anpassungsfähigkeit des Herz-Kreislauf-Systems auf Störungen zu untersuchen.

Die Aufgabe wird erfindungsgemäß dadurch gelöst, dass während einer Erhöhung des Gefäßwiderstandes mindestens eines Gefäßes - z.B. durch Abbinden eines Armes mit Hilfe einer Blutdruck-Manschette - ein EKG gemessen wird und die Veränderungen im EKG aufgrund des veränderten Gefäßwiderstandes gemessen werden.

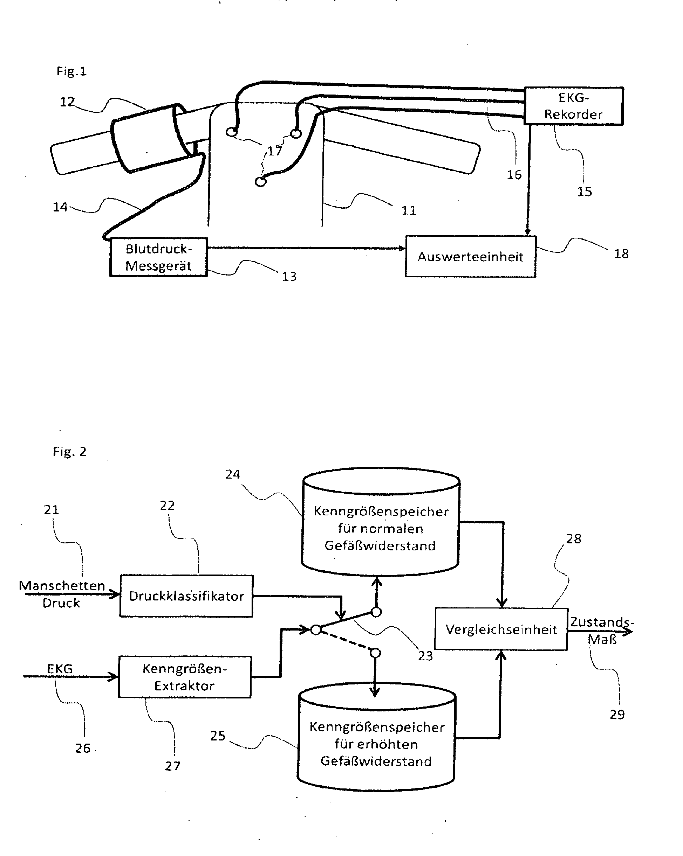

Eine mögliche Ausführungsform, die das erfundene Verfahren anwendet, ist in Fig. 1 dargestellt. Einer Person (11) wird dabei ein Arm mit Hilfe einer Blutdruck-Manschette (12) abgebunden. Die Steuerung der Blutdruckmanschette erfolgt mit einem adaptierten Blutdruckrnessgerät (13), welches mit einem Luftschlauch (14) mit der Manschette verbunden ist. Die Messung des EKGs erfolgt mit einem EKG-Rekorder (15), der über Kabel (16) mit am Körper der Person angebrachten Elektroden (17) verbunden ist. Die Daten des Blutdruckmessgeräts und des EKG-Rekorders werden in einer Auswerteeinheit (18) analysiert, um die Eigenschaften des Herz-Kreislauf-Systems und/oder die Anpassungsfähigkeit des Herz-Kreislauf-Systems auf Störungen zu untersuchen.

Eine mögliche Ausführungsform der Auswerteeinheit ist in Fig. 2 dargestellt. Der Manschettendruck (21) wird vom Blutdruckmessgerät an die Auswerteeinheit übergeben und einem Druckklassifikator (22) zugeführt, welcher einen Umschalter (23) je nach Ergebnis der Klassifikation auf verschiedene Speicher stellt, z.B. entweder auf einen Speicher für Kenngrößen bei normalem Gefäßwiderstand (24) oder auf einen Speicher für Kenngrößen bei erhöhtem Gefäßwiderstand (25). Im einfachsten Fall kann der Druckklassifikator den Manschettendruck mit einem Schwellwert vergleichen. Die Kenngrößen selbst werden aus dem EKG (26), welches vom EKG-Rekorder an die Auswerteeinheit übertragen wird, in einem Kenngrößen-Extraktor (27) berechnet. Als Kenngröße kann z.B. die QRS-Amplitude, die QRS-Dauer, die maximale oder minimale Steigung der R-Zacke etc. dienen. Die berechneten und in den beiden Speichern abgelegten Kenngrößen werden schließlich in einer Vergleichseinheit (28) miteinander verglichen. Als Ergebnis wird ein Zustandsmaß (29) ausgegeben, welches den Zustand des Herz-Kreislauf-Systems und seiner Anpassungsfähigkeit auf Störungen beschreibt. Die Vergleichseinheit kann dabei im einfachsten Fall die Differenz des Mittelwertes einer Kenngröße aus den beiden Speichern als Zustandsmaß zurückgeben, sie kann aber auch andere Vergleiche der Zeitverläufe von einer oder mehreren Kenngrößen durchführen.

Da die Änderungen, die durch das Erhöhen des Gefäßwiderstandes im EKG verursacht werden, nur von geringem Ausmaß sein können, kann eine Mittelung von mehreren Signalen sinnvoll sein, um den Einfluss von Störgrößen auf das Ergebnis zu vermindern. Die Mittelung kann sich einerseits auf mehrere Herzschläge beziehen (z.B. Mittel von 10 Herzschlägen vor dem Abschnüren verglichen mit dem mittel von 10 Herzschlägen nach dem Abschnüren), oder auch auf mehrere vollständige Messzyklen (10 maliges Erhöhen des Gefäßwiderstandes). Alternativ zur Mittelung können dabei auch andere Verfahren herangezogen werden, z.B. die Bestimmung anderer statistischer Parameter wie die Standardabweichung etc.

Je nachdem, welche Gefäße von der Erhöhung des Gefäßwiderstandes betroffen sind, können aus dem gemessenen EKG unterschiedliche Aussagen getroffen werden. Sowohl die Wahl, welches Gefäß abgeschnürt wird, als auch die genaue Stelle, an der das Gefäß abgeschnürt wird, hat Auswirkungen auf das Herz-Kreislauf-System und damit auf das EKG. Es können auch mehrere Gefäße gleichzeitig abgeschnürt werden. Dadurch können die Ursachen der beobachteten Effekte einerseits auf bestimmte Gefäße eingeschränkt werden, andererseits wird auch die Last-Veränderung für das Herz verändert, wenn z.B. zwei Extremitäten gleichzeitig abgeschnürt werden, wodurch die Änderungen im EKG verstärkt werden können.

Durch die Veränderung des Gefäßwiderstandes wird die Kraft verändert, die das Herz zum Auswerfen des Blutes benötigt. Der Füllzustand des Herzens, damit seine Geometrie und schließlich die Erregung und Kontraktion des Herzens verändern sich. Diese Änderungen können im EKG gemessen werden. Mögliche Änderungen im EKG finden sich in der Breite, Höhe, Steigung, Krümmung etc. des QRS-Komplexes oder eines anderen Abschnittes des EKGs. Je nach Zustand der Gefäße und je nach Anpassungsfähigkeit des Herz-Kreis-Systems ist dieser Effekt deutlicher oder weniger deutlich zu sehen, dauert länger oder kürzer an, etc.

Die Veränderung des Gefäßwiderstandes verändert nicht nur Kenngrößen des EKGs, sondern auch andere physiologische Parameter wie Blutmenge in den Abgeschnürten Gefäßen, damit verbunden deren Impedanz, die Sauerstoffsättigung im Blut, die Blutdurchflussgeschwindigkeit, etc. Es können daher auch andere Parameter in die Berechnung inkludiert werden, um einerseits das Ausmaß der Störung des Regelkreises zu bestimmen (Eingangsgrößen), andererseits aber auch um die Reaktion des Regelsystems auf die Störung zu quantifizieren (Ausgangsgrößen).

## Patentansprüche

1. Verfahren zur Beurteilung des kardiovaskulären Status einer Person, insbesondere der Eigenschaften der Arterien und der Anpassungsfähigkeit des Herzens, durch Bestimmung spezifischer Kenngrößen des EKGs, **dadurch gekennzeichnet,**
**dass** während der EKG-Messung der Gefäßwiderstand mindestens eines Blutgefäßes dieser Person künstlich erhöht wird und
**dass** die Kenngrößen des EKGs bei erhöhtem und bei normalem Gefäßwiderstand miteinander verglichen werden.

2. Verfahren nach Anspruch 1, **dadurch gekennzeichnet,**
**dass** der Gefäßwiderstand mehrmals im selben oder in variierendem Ausmaß erhöht wird und die Kenngrößen der verschiedenen Unterbrechungen und/oder ihre Veränderung und/oder ein aus den Kenngrößen der unterschiedlichen Messungen zusammengefasster statistischer Parameter, insbesondere der Mittelwert und/oder die Standardabweichung, und/oder ein Vergleich des EKG-Verlaufs mehrerer Herzschläge zur Berechnung herangezogen wird, wobei sowohl das EKG-Signal vor der Bestimmung der Kenngrößen über mehrere Herzschläge gemittelt werden kann, als auch die Kenngrößen mehrerer Herzschläge oder die Vergleichsmaße zwischen den Kenngrößen mehrerer Herzschläge gemittelt werden können.

3. Verfahren nach einem der Ansprüche 1 bis 2, **dadurch gekennzeichnet,**
**dass** die Erhöhung des Gefäßwiderstandes an einer oder mehreren Extremitäten durchgeführt wird, wobei die Erhöhung des Gefäßwiderstandes auch an unterschiedlich weit distalen Stellen erfolgen kann und der Einfluss des Abstandes auf spezifische Kenngrößen des EKGs in die Bewertung einfließt.

4. Verfahren nach einem der Ansprüche 1 bis 3, **dadurch gekennzeichnet,**
**dass** gleichzeitig mit der Bestimmung spezifischer Kenngrößen des EKGs auch spezifische Kenngrößen des Blutdrucks bestimmt werden und in die Bewertung einfließen, wobei die Blutdruckmessung an mehreren Stellen simultan erfolgen kann und die einzelnen Ergebnisse in die Bewertung einfließen.

5. Verfahren nach einem der Ansprüche 1 bis 4, **dadurch gekennzeichnet,**
**dass** als Kenngröße des EKGs die Amplitude und/oder die Breite und/oder der Abstand und/oder die Steigung und/oder die Krümmung von bestimmten Abschnitten des EKGs herangezogen wird und/oder die Ähnlichkeit von bestimmten Abschnitten des EKGs mit vordefinierten Formen und/oder mit anderen Abschnitten des EKGs und/oder mit aus mehreren Herzschlägen gemittelten Abschnitten des EKGs und/oder die Konstanz und/oder Variabilität und/oder die Art des Einschwingvorgangs nach Erhöhung und/oder nach Wiederherstellen des Gefäßwiderstandes in den Extremitäten und/oder ein Vergleich des Einschwingvorganges nach Erhöhung und nach Wiederherstellen des Gefäßwiderstandes einer oder mehrerer Kenngrößen.

6. Verfahren nach einem der Ansprüche 1 bis 5, **dadurch gekennzeichnet,**
**dass** gleichzeitig zur EKG-Messung auch der Einfluss der Erhöhung oder Wiederherstellung des Gefäßwiderstandes auf andere Messgrößen, insbesondere Blut-Durchflussgeschwindigkeit, Sauerstoffsättigung und/oder Gewebeimpedanz, untersucht wird.

7. Anordnung zur Beurteilung des kardiovaskulären Status einer Person, insbesondere der Eigenschaften der Arterien und der Anpassungsfähigkeit des Herzens, durch Bestimmung spezifischer Kenngrößen des EKGs, **dadurch gekennzeichnet,**
**dass** während der EKG-Messung mit Hilfe einer Gefäßwiderstand-Erhöh-Einheit der Gefäßwiderstand mindestens eines Blutgefäßes dieser Person künstlich erhöht wird und
**dass** in einer Vergleichseinheit die Kenngrößen des EKGs bei erhöhtem und bei normalem Gefäßwiderstand miteinander verglichen werden.

8. Anordnung nach Anspruch 7, **dadurch gekennzeichnet,**
**dass** die Gefäßwiderstand-Erhöh-Einheit den Gefäßwiederstand mehrmals im selben oder in variierendem Ausmaß erhöht und die Vergleichseinheit die Kenngrößen der verschiedenen Unterbrechungen und/oder ihre Veränderung und/oder ein aus den Kenngrößen der unterschiedlichen Messungen zusammengefasster statistischer Parameter, insbesondere der Mittelwert und/oder die Standardabweichung, und/oder ein Vergleich des EKG-Verlaufs mehrerer Herzschläge zur Berechnung heranzieht, wobei sie sowohl das EKG-Signal vor der Bestimmung der Kenngrößen über mehrere Herzschläge, als auch die Kenngrößen mehrerer Herzschläge oder die Vergleichsmaße zwischen den Kenngrößen mehrerer Herzschläge mitteln kann.

9. Anordnung nach einem der Ansprüche 7 bis 8, **dadurch gekennzeichnet,**
**dass** Gefäßwiderstand-Erhöh-Einheiten an einer oder mehreren Extremitäten angebracht werden, wobei sie auch an unterschiedlich weit distalen Stellen liegen können und der Vergleichseinheit auch der Abstand der Einheiten zum Torso als Eingangsgröße zur Verfügung gestellt wird.

10. Anordnung nach einem der Ansprüche 7 bis 9, **dadurch gekennzeichnet,**
**dass** die Gefäßwiderstand-Erhöh-Einheit durch mindestens ein Blutdruckmessgerät realisiert wird, wobei die Blutdruckmessung an mehreren Stellen simultan erfolgen kann und dass der Vergleichseinheit auch der Verlauf des Blutdrucks an einer oder mehreren Stellen als Eingangsgröße zur Verfügung gestellt wird.

11. Anordnung nach einem der Ansprüche 7 bis 10, **dadurch gekennzeichnet,**
**dass** zumindest eine der Elektroden zur Messung des EKGs in die Gefäßwiderstand-Erhöh-Einheit integriert wird.

12. Anordnung nach einem der Ansprüche 7 bis 11, **dadurch gekennzeichnet,**
**dass** die Vergleichseinheit als Kenngröße des EKGs die Amplitude und/oder die Breite und/oder der Abstand und/oder die Steigung und/oder die Krümmung von bestimmten Abschnitten des EKGs berechnet und/oder die Ähnlichkeit von bestimmten Abschnitten des EKGs mit vordefinierten Formen und/oder mit anderen Abschnitten des EKGs und/oder mit aus mehreren Herzschlägen gemittelten Abschnitten des EKGs und/oder die Konstanz und/oder Variabilität und/oder die Art des Einschwingvorgangs nach Erhöhung und/oder nach Wiederherstellen des Gefäßwiderstandes in den Extremitäten und/oder ein Vergleich des Einschwingvorganges nach Erhöhung und nach Wiederherstellen des Gefäßwiderstandes einer oder mehrerer Kenngrößen..

13. Anordnung nach einem der Ansprüche 7 bis 12, **dadurch gekennzeichnet,**
**dass** weitere Sensoren an der Person angebracht werden, die gleichzeitig zur EKG-Messung auch den Einfluss der Erhöhung oder Wiederherstellung des Gefäßwiderstandes auf andere Messgrößen, insbesondere Blut-Durchflussgeschwindigkeit, Sauerstoffsättigung und/oder Gewebeimpedanz, untersuchen.
